(19) Europäisches Patentamt
European Patent Office
Office européen des brevets

(11) EP 0 937 076 B1

(12) EUROPEAN PATENT SPECIFICATION

(45) Date of publication and mention of the grant of the patent:
**14.08.2002 Bulletin 2002/33**

(21) Application number: **97913743.7**

(22) Date of filing: **30.10.1997**

(51) Int Cl.[7]: **C07D 471/10**, A61K 31/435, C07D 495/10, C07D 221/20, C07D 295/12, C07D 249/08, C07D 257/04, C07D 213/63, C07D 239/48, C07D 233/61, C07D 473/18

(86) International application number:
**PCT/US97/19164**

(87) International publication number:
**WO 98/20010 (14.05.1998 Gazette 1998/19)**

# (54) SUBSTITUTED OXIMES, HYDRAZONES AND OLEFINS AS NEUROKININ ANTAGONISTS

SUBSTITIERTE OXIME, HYDRAZONE UND OLEFINE ALS NEUROKININ ANTAGONISTEN

OXIMES, HYDRAZONES ET OLEFINES SUBSTITUEES UTILISEES COMME ANTAGONISTES DES NEUROKININES

(84) Designated Contracting States:
**AT BE CH DE DK ES FI FR GB GR IE IT LI LU NL PT SE**
Designated Extension States:
**LT LV RO**

(30) Priority: **01.11.1996 US 742538**

(43) Date of publication of application:
**25.08.1999 Bulletin 1999/34**

(73) Proprietor: **SCHERING CORPORATION**
**Kenilworth, New Jersey 07033-0530 (US)**

(72) Inventors:
- **SHIH, Neng-Yang**
  **North Caldwell, NJ 07006 (US)**
- **REICHARD, Gregory, A.**
  **Morris Plains, NJ 07950 (US)**
- **ASLANIAN, Robert, G.**
  **Rockaway, NJ 07866 (US)**

(74) Representative: **Ritter, Stephen David et al**
**Mathys & Squire**
**100 Gray's Inn Road**
**London WC1X 8AL (GB)**

(56) References cited:
**US-A- 5 434 158**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### BACKGROUND OF THE INVENTION

The present invention relates to a genus of substituted oximes, hydrazones and olefins useful as antagonists of tachykinin receptors, in particular as antagonists of the neuropeptides neurokinin-1 receptor ($NK_1$) and/or neurokinin-2 receptor ($NK_2$) and/or neurokinin-3 receptor ($NK_3$).

Neurokinin receptors are found in the nervous system and the circulatory system and peripheral tissues of mammals, and therefore are involved in a variety of biological processes. Neurokinin receptor antagonists are consequently expected to be useful in the treatment or prevention of various mammalian disease states, for example asthma, cough, bronchospasm, inflammatory diseases such as arthritis, central nervous system conditions such as migraine and epilepsy, nociception, and various gastrointestinal disorders such as Crohn's disease.

In particular, $NK_1$ receptors have been reported to be involved in microvascular leakage and mucus secretion, and $NK_2$ receptors have been associated with smooth muscle contraction, making $NK_1$ and $NK_2$ receptor antagonists especially useful in the treatment and prevention of asthma.

Some $NK_1$ and $NK_2$ receptor antagonists have previously been disclosed: arylalkylamines were disclosed in U.S. Patent 5,350,852, issued September 27, 1994, spiro-substituted azacycles were disclosed in WO 94/29309, published December 22, 1994, and in WO 94/17045, published August 4, 1994; substituted aryl piperazines were disclosed in WO 96/10568, published April 11, 1996.

### SUMMARY OF THE INVENTION

Compounds of the present invention are represented by the formula I

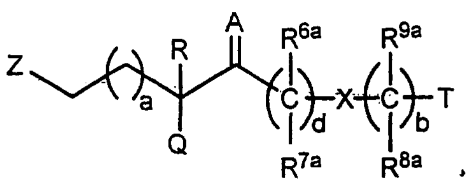

I

or a pharmaceutically acceptable salt thereof, wherein:

a is 0, 1, 2 or 3;
b and d are independently 0, 1 or 2;
R is H;
A is $=N-OR^1$;
X is a bond, -C(O)-, -O-, $-NR^6-$, $-S(O)_e-$, $-N(R^6)C(O)-$, $-OC(O)NR^6-$, $-C(=NOR^1)-$, provided that when d is 0, X is a bond, -C(O)-, $-NR^6-$, $-N(R^6)C(O)-$, $-OC(O)NR^6-$, $-C(=NOR^1)-$;
T is $R^4$-phenyl;
Q is $R^5$-phenyl;
$R^1$ is H, $C_{1-6}$ alkyl, $-(CH_2)_n-G$, $-(CH_2)_p-M-(CH_2)_n$ -G, or $-C(O)N(R^6)(R^7)$;
$R^4$ and $R^5$ are independently 1-3 substituents independently selected from the group consisting of H, halogeno, $-OR^6$, $C_{1-6}$ alkyl, $-CF_3$;
$R^6$, $R^7$,and $R^8$, are independently selected from the group consisting of H, $C_{1-6}$ alkyl, $C_2$-$C_6$ hydroxyalkyl, $C_1$-$C_6$ alkoxy-$C_1$-$C_6$ alkyl, $R^{15}$-phenyl, and $R^{15}$-benzyl; or $R^6$ and $R^7$, together with the nitrogen to which they are attached, form a ring of 5 to 6 members, wherein 0, 1 or 2 ring members are selected from the group consisting of -O-, -S- and $-N(R^{19})-$;
$R^{6a}$, $R^{7a}$, and $R^{8a}$ are independently selected from the group consisting of H, $C_2$-$C_6$ hydroxyalkyl, and $C_1$-$C_6$ alkoxy-$C_1$-$C_6$ alkyl;
$R^9$ and $R^{9a}$ are independently selected from the group consisting of $R^6$;
$R^{10}$ and $R^{10a}$ are independently selected from the group consisting of H and $C_{1-6}$ alkyl;
$R^{15}$ is 1 to 3 substituents independently selected from the group consisting of H, $C_1$-$C_6$ alkyl, $C_1$-$C_6$ alkoxy, $C_1$-$C_6$ alkylthio, halogeno, $-CF_3$, $-C_2F_5$, $-COR^{10}$, $-CO_2R^{10}$, $-C(O)N(R^{10})_2$, $-S(O)_eR^{10a}$, -CN, $-N(R^{10})COR^{10}$, $-N(R^{10})CON(R^{10})_2$ and $-NO_2$;
$R^{19}$ is H, $C_1$-$C_6$ alkyl, $-C(O)N(R^{10})_2$, $-CO_2R^{10}$, $-(C(R^8)(R^9))_f-CO_2R^{10}$ or $-(C(R^8)(R^9))_u-C(O)N(R^{10})_2$;
f, n and p are independently 1-6;

u is 0-6;

G is selected from the group consisting of $R^4$-heteroaryl, $-OR^6$, $-CO_2R^6$, $-CON(R^7)(R^9)$, $-C(=NOR^8)N(R^6)(R^7)$, and $-C(O)N(R^9)-(R^4$-heteroaryl), provided that when n is 1 and u is 0, G is not -OH;

M is selected from the group consisting of -O- and $-C(O)N(R^9)-$, provided that when n is 1, G is not OH;

e is 0, 1 or 2, provided that when e is 1 or 2, $R^{10a}$ is not H;

Z is

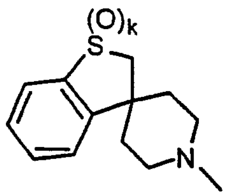 , 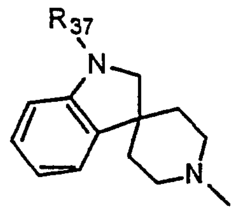 , or 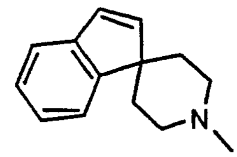 ,

$R^{37}$ is selected from a group consisting of:

(1) H,
(2) $C_{1-8}$ linear or branched alkyl, unsubstituted, monosubstituted or multiply substituted with $-OR^{35}$, =O, $-NHCOR^{35}$, $-NR^{35}R^{36}$, -CN, -halogeno,$-CF_3$, -phenyl or substituted phenyl, wherein the substitutents on phenyl are selected from the group consisting of -OH, -CN, halogeno and $-CF_3$;
(3) $S(O)R^{38}$, wherein $R^{38}$ is $C_{1-6}$ linear or branched alkyl, unsubstituted, mono di or trisubstituted with a substituent selected from the group consisting of =O, -CN, $-OR^{35}$, $-NR^{35}R^{36}$, $-NR^{36}OR^{36}$,-halogeno,$-CF_3$, -phenyl or mono, di or trisubstituted phenyl, wherein the substituents on the phenyl are independently selected from the group consisting of -OH, =O, -CN, $-NR^{35}R^{36}$, $-NR^{35}COR^{36}$, -halogeno, $-CF_3$ and $C_{1-3}$ alkyl;
(4) $SO_2R^{38}$,
(5) $COR^{38}$,
(6) $CO_2R^{38}$;
(7) $CONR^{36}R^{38}$;

$R^{35}$ and $R^{36}$ are independently selected from:

(a) H,
(b) $C_{1-6}$ alkyl, or mono or disubstituted $C_{1-6}$ alkyl, wherein the substitutents independently selected from the group consisting of phenyl, unsubstituted or substituted with -OH, $C_{1-3}$ alkyl, -CN, halogeno, $-CF_3$ or $C_{1-4}$ alkoxy; -OH; =O; -CN; halogeno; or $-CF_3$;
(c) phenyl, pyridinyl or thiophene, or mono, di or trisubstituted phenyl, pyridinyl or thiophene, wherein the substitutents are independently selected from the group consisting of -OH, $C_{1-4}$ alkyl, -CN, halogeno and $-CF_3$;
(d) $C_{1-3}$ alkyloxy,

or

$R^{35}$ and $R^{36}$ are joined together to form a 5-, 6-, or 7-membered monocyclic saturated ring containing 1 or 2 heteroatoms independently selected from nitrogen, oxygen, and sulfur, and in which the ring is unsubstituted or mono or disubstituted, wherein the substituents are independently selected from the group consisting of -OH, =O, -CN, halogeno and $-CF_3$.

Preferred compounds are of the formula I wherein X is -O-, $-NR^6-$, $-N(R^6)C(O)$-or $-OC(O)NR^6$. Additional preferred definitions are: b is 1 or 2 when X is -O- or $-NR^6-$; b is 0 when X is $-N(R^6)C(O)-$; and d is 1 or 2. Compounds wherein $R^{6a}$, $R^{7a}$, $R^{8a}$ and $R^{9a}$ are hydrogen are also preferred. Especially preferred are compounds wherein $R^{8a}$ and $R^{9a}$ are each hydrogen, d and b are each 1, X is -O-, $-NR^6-$, $-N(R^6)C(O)-$ or $-OC(O)NR^6$, T is $R^4$-aryl and $R^4$ is two substituents selected from $C_1$-$C_6$ alkyl, halogeno, $-CF_3$ and $C_1$-$C_6$ alkoxy.

A preferred definition for Q is $R^5$-phenyl, wherein $R^5$ is preferably two halogeno substituents.

Preferred definitions of Z are exemplified by the following structures:

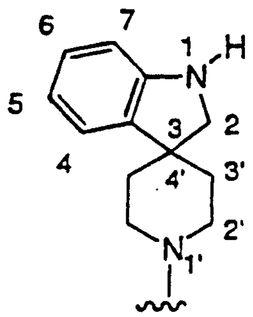
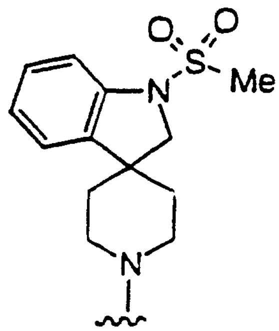
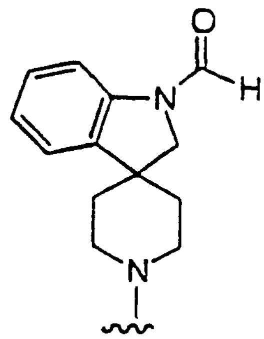

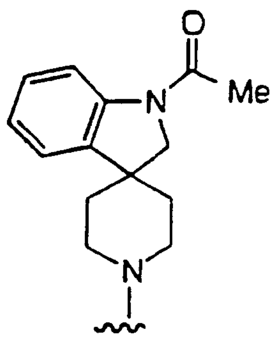
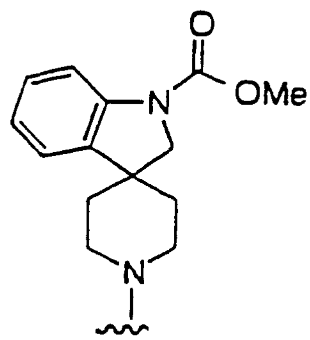
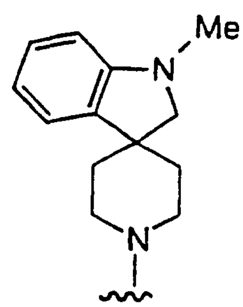

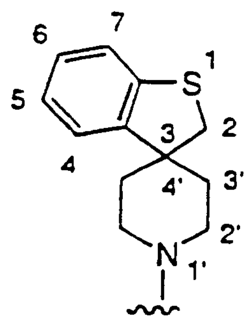
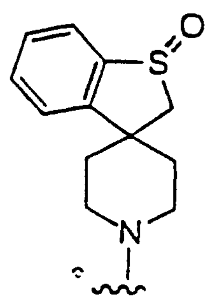
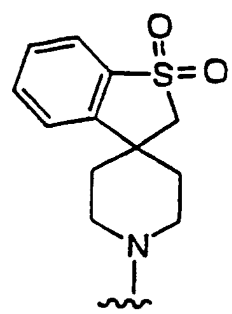

This invention also relates to the use of a compound of formula I in the treatment of asthma, cough, bronchospasm, inflammatory diseases such as arthritis, central nervous system conditions such as migraine and epilepsy, nociception, and various gastrointestinal disorders such as Crohn's disease.

In another aspect, the invention relates to a pharmaceutical composition comprising a compound of formula I in a pharmaceutically acceptable carrier. The invention also relates to the use of said pharmaceutical composition in the treatment of asthma, cough, bronchospasm, inflammatory diseases such as arthritis, migraine, nociception, and various gastrointestinal disorders such as Crohn's disease.

DETAILED DESCRIPTION

As used herein, the term "alkyl" means straight or branched alkyl chains. "Lower alkyl" refers to alkyl chains of 1-6 carbon atoms and, similarly, lower alkoxy refers to alkoxy chains of 1-6 carbon atoms.

"Cycloalkyl" means cyclic alkyl groups having 3 to 6 carbon atoms. "Bridged cycloalkyl" refers to $C_7$-$C_{10}$ saturated rings comprised of a cycloalkyl ring or a fused bicycloalkyl ring and an alkylene chain joined at each end to non-adjacent carbon atoms of the ring or rings. Examples of such bridged bicycloalkyl rings are adamantyl, myrtanyl, noradamantyl, norbornyl, bicyclo[2.2.1]heptyl, 6,6-dimethylbicyclo[3.1.1]heptyl, bicyclo[3.2.1]octyl, and bicyclo[2.2.2]octyl.

"Aryl" means phenyl, naphthyl, indenyl, tetrahydronaphthyl, indanyl, anthracenyl or fluorenyl.

"Halogeno" refers to fluoro, chloro, bromo or iodo atoms.

"Heterocycloalkyl" refers to 4- to 6-membered saturated rings comprising 1 to 3 heteroatoms independently selected from the group consisting of -O-, -S- and -N($R^{19}$)-, with the remaining ring members being carbon. Examples of heterocycloalkyl rings are tetrahydrofuranyl, pyrrolidinyl, piperidinyl, morpholinyl, thiomorpholinyl and piperazinyl. $R^4$-heterocycloalkyl refers to such groups wherein substitutable ring carbon atoms have an $R^4$ substituent.

"Heteroaryl" refers to 5- to 10-membered single or benzofused aromatic rings comprising 1 to 4 heteroatoms independently selected from the group consisting of -O-, -S- and -N=, provided that the rings do not include adjacent oxygen and/or sulfur atoms. Examples of single-ring heteroaryl groups are pyridyl, isoxazolyl, oxazolyl, oxadiazolyl, furanyl, pyrrolyl, thienyl, imidazolyl, pyrazolyl, tetrazolyl, thiazolyl, isothiazolyl, thiadiazolyl, pyrazinyl, pyrimidyl, pyridazinyl and triazolyl. Examples of benzofused heteroaryl groups are indolyl, quinolyl, thianaphthenyl (i.e., benzothienyl) benzimidazolyl, benzoxazolyl, and benzofurazanyl. N-oxides of nitrogen-containing heteroaryl groups are also included. All positional isomers are contemplated, e.g., 1-pyridyl, 2-pyridyl, 3-pyridyl and 4-pyridyl. $R^4$-heteroaryl refers to such groups wherein substitutable ring carbon atoms have an $R^4$ substituent.

Where $R^6$ and $R^7$ substituents on a nitrogen atom form a ring and additional heteroatoms are present, the rings do not include adjacent oxygen and/or sulfur atoms or three adjacent heteroatoms. Typical rings so formed are morpholinyl, piperazinyl and piperidinyl.

In the above definitions, wherein variables $R^6$, $R^7$, $R^8$, $R^9$, $R^{10}$, and $R^{15}$, for example, are said to be independently selected from a group of substituents, we mean that $R^6$, $R^7$, $R^8$, $R^9$, $R^{10}$, and $R^{15}$ are independently selected, but also that where an $R^6$, $R^7$, $R^8$, $R^9$, $R^{10}$, or $R^{15}$ variable occurs more than once in a molecule, those occurrences are independently selected. Similarly, $R^4$ and $R^5$ can be independently selected from a group of substituents, and where more than one $R^4$ and $R^5$ are present, the substitutents are independently selected; those skilled in the art will recognize that the size and nature of the substituent(s) will affect the number of substituents which can be present.

Compounds of formula I can have at least one asymmetrical carbon atom and all isomers, including diastereomers, enantiomers and rotational isomers, as well as E and Z isomers of the oxime, hydrazone and olefin groups, are contemplated as being part of this invention. The invention includes d and l isomers in both pure form and in admixture, including racemic mixtures. Isomers can be prepared using conventional techniques, either by reacting optically pure or optically enriched starting materials or by separating isomers of a compound of formula I.

Those skilled in the art will appreciate that for some compounds of formula I, one isomer will show greater pharmacological activity than other isomers.

Compounds of the invention have at least one amino group which can form pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds of the invention are acidic (e.g., those compounds which possess a carboxyl group). These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine and the like.

Compounds of formula I can be prepared using methods well known to those skilled in the art. Following are typical procedures for preparing various compounds; the skilled artisan will recognize that other procedures may be applicable, and that the procedures may be suitably modified to prepare other compounds within the scope of formula I.

Procedure A:

Compounds of formula I wherein R is H, a and d are each 1, X is -O- or -S- and the remaining variables are as defined above (Q is exemplified as $R^5$-phenyl, but the procedure is not limited to this definition) can be prepared according to the following reaction scheme:

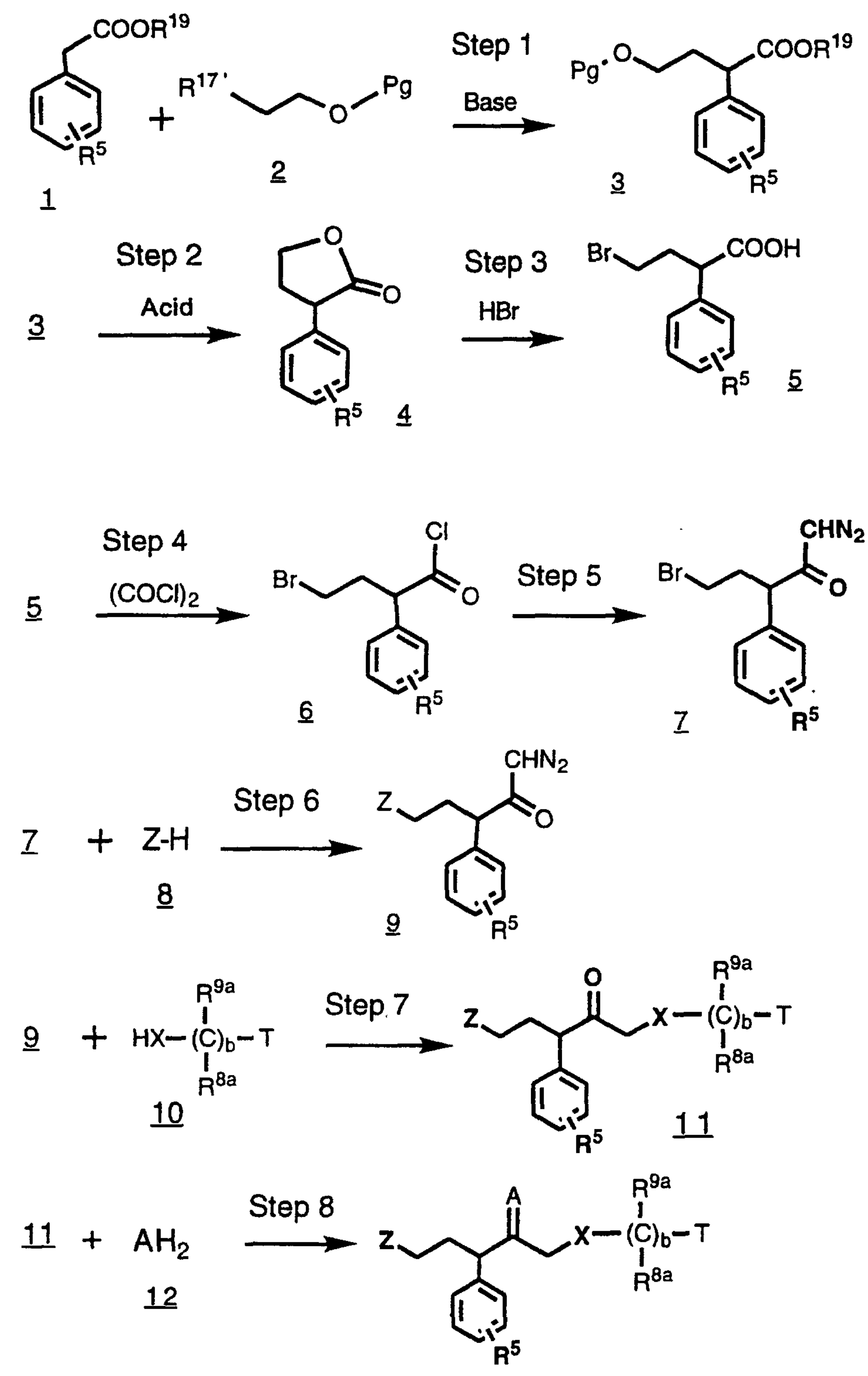


In step 1, the ester (preferably methyl) of the substituted aryl acetic acid of formula 1, wherein $R^{19}$ is a lower alkyl group, preferably methyl, is reacted with a compound of formula 2, wherein $R^{17'}$ is a leaving group such as Br, I or triflate, and Pg is a suitable protecting group such as tetrahydropyranyl, and a base to prepare a compound of formula 3. The base can be chosen from any strong base including LDA or lithium bis(trimethylsilyl)amide. The reaction is carried out in an inert organic solvent such as THF at temperatures of -15 to about 65°C.

In step 2, a compound of formula 3 is reacted with an acid in a solvent such as $CH_3OH$, at temperatures ranging from -10 to 65°C. The acid need not to be used in stochiometric amount. Alternatively, a compound of formula 4 can be prepared directly from step 1 without isolating the compound of formula 3: the reaction mixture obtained after the work up of the reaction described in step 1 can be dissolved in the solvent and reacted with the acid.

In step 3, a compound of formula 4 is reacted with an acid such hydrobromic acid (HBr) dissolved in a suitable solvent such as acetic acid. The reaction is performed at temperatures ranging from 5 to 45°C.

In step 4, the carboxylic acid of formula 5 is reacted with a halogenating agent such as $SOCl_2$ or $(COCl)_2$ in an appropriate solvent such $CH_2Cl_2$ to form the acid halide of formula 6.

In step 5, the compound of formula 6 is reacted with an alkylating agent such as diazomethane to obtain the compound of formula 7. This reaction may be performed at temperatures lower than ambient using an appropriate solvent such as $Et_2O$.

In step 6, a compound of formula 7 is reacted with a compound of formula 8 to obtain a compound of formula 9. The reaction is carried out in a suitable solvent, e.g. EtOAc, at temperatures below 85°C. Bases such as $Et_3N$ may be beneficial to the reaction.

In step 7, a compound of formula 9 is reacted with a compound of formula 10, wherein X is -O- or -S-, T is H, $R^4$-aryl, $R^4$-cycloalkyl, $R^4$-alkyl, $R^4$-bicyclo or tricycloalkyl, and $R^{8a}$, $R^{9a}$, b and $R^4$ are as defined above in an appropriate solvent, e.g. $CH_2Cl_2$, with a Lewis acid, e.g. $BF_3$, at temperatures lower than 50°C.

In step 8 a compound of formula 11 is reacted with a compound of formula 12, wherein A is as defined above, in a solvent such as pyridine, to obtain the desired product.

Procedure B:

Compounds of formula I, wherein the variables are as defined above, can be prepared as shown in the following reaction scheme:

Step 1:

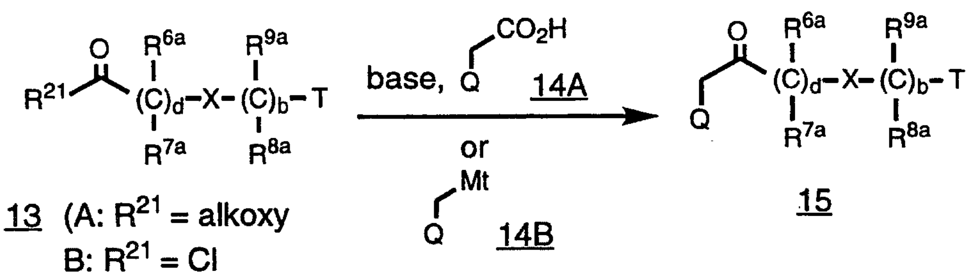


14A

14B

15

13 (A: $R^{21}$ = alkoxy
B: $R^{21}$ = Cl
C: $R^{21}$ = $-N(CH_3)OCH_3$)

In step 1, a compound of formula 14A, wherein Q is as defined above, is reacted with a base such as lithium disopropylamide (LDA) or KH in an inert organic solvent such at THF or DME to generate a dianion. An acid chloride, ester or amide of formula 13A, 13B, or 13C is added to give a ketone of formula 15. Preferable reaction temperatures ranges from -78°C to 30°C.

Alternatively, compounds of formula 15 can be generated by the reaction of a compound of formula 13, preferably 13C, with a metallated species of formula $QCH_2Mt$ where Mt is a metal, such as MgHal, wherein "Hal" is halogen, or lithium. The metallated species $QCH_2Mt$ can be generated by conventional procedures, such as treatment compounds of formula $QCH_2Hal$ with Mg or by treating $QCH_3$ with an organolithium base.

Step 2:

15

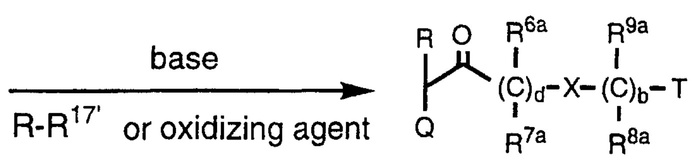


16

In step 2, for compounds of formula I wherein R is not hydrogen, the ketone 15 is reacted with a suitable

base, such as LDA or KH in an inert organic solvent such as THF. For compounds wherein R is alkyl or hydroxyalkyl, a compound R-$R^{17'}$, wherein $R^{17'}$ (as defined above) is added. For compounds wherein R is OH, an appropriate oxidizing agent such as dimethyldioxirane or Davis reagent is added. Preferable reaction temperatures range from -78° to 50°C.

Step 3:

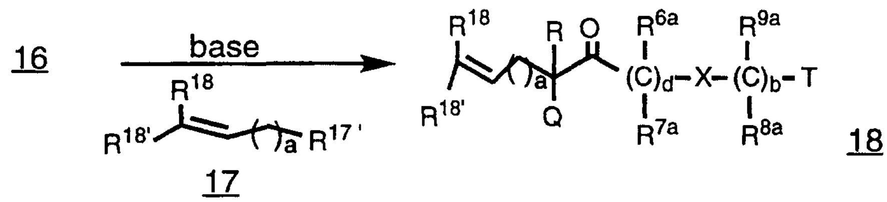


In step 3, ketone 16 is reacted with a base such as LDA in a solvent such as THF, then an olefin of formula 17 is added, wherein $R^{18}$ and $R^{18'}$ are independently H or lower alkyl and $R^{17'}$ is as defined above, to give the adduct 18. Preferable reaction temperatures range from -78 to 60°C.

Step 4:

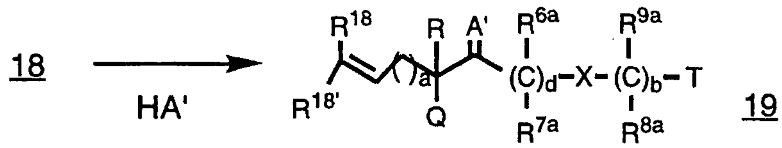


In step 4, ketone 18 is reacted with HA', wherein A' is NH-$OR^1$, NH-N($R^2$)($R^3$) or $NHR^{26}$, in an organic solvent such as pyridine at a temperature from 25°C to 150°C to give a compound of formula 19.

Step 5:

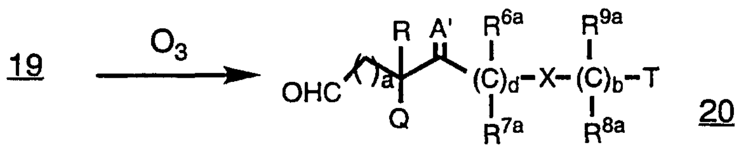


In step 5, a compound of formula 19 is oxidized by ozonolysis to give an aldehyde of formula 20. Suitable organic solvents include EtOAc, ethanol or the like. Preferable reaction temperatures are from -78 to 0°C.

Step 6:

20
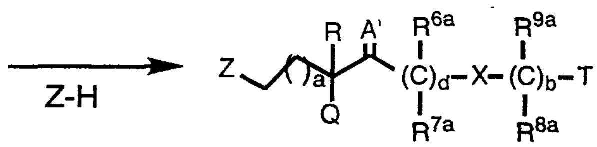


In step 6, an aldehyde of formula 20 is reacted with a compound of formula Z-H, wherein Z is a suitably substituted amine (as its acid salt e.g. HCl or maleate or as its free base) and a hydride source such as $NaBH_3CN$ or sodium triacetoxyborohydride in a protic solvent (e.g. $CH_3OH$, $CH_3CH_2OH$, or $CF_3CH_2OH$) with 3A sieves. Any suitable temperature can be used with preferable temperatures being between 0°C and 25°C.

Alternatively, a compound of formula I can be prepared from 18 by the following reaction scheme:

18
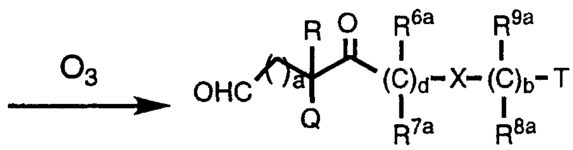

21

Compound 18 is oxized to a compound of formula 21 under conditions similar to those described for step 5 above. The aldehyde of formula 21 is reacted with a compound of formula Z-H in a manner similar to that described in Step 6, and the resultant ketone is then reacted with a compound of the formula HA' as described above in Step 4 to obtain the compound of formula I.

Procedure C:

Step 1:

16
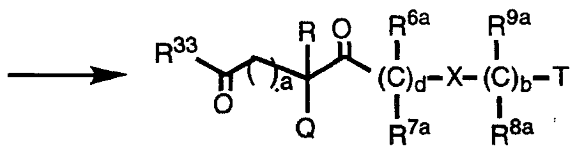
23

In step 1, compounds of formula 16 treated with an appropriate base, such as NaH, are reacted with alkylating agents of the formula $R^{33}C(O)CH_2R^{17}$ or $R^{33}C(O)CH=CH_2$ wherein $R^{33}$ is alkoxy or $-N(CH_3)OCH_3$ and $R^{17}$ is a leaving group such as Cl or Br.

Step 2:

Step 2:

23
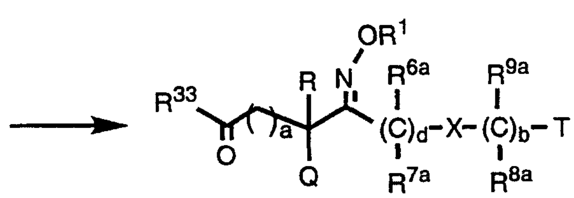
24

In step 2, compounds of formula 23 can be converted to the corresponding oxime of formula 24 in a manner

similar to that described in Procedure B, Step 4.

Step 3:

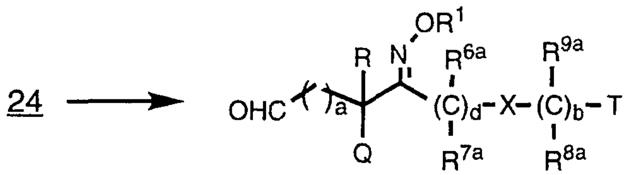

24 25

In step 3, compounds of formula 24 (or 23, i.e., wherein A' is O) are converted to the corresponding aldehyde 25 (or lactol from the keto-ester 23) by treatment with a suitable reducing agent such a DIBAL, in an suitable inert organic solvent such as THF, at a temperature from about -100 to -20°C.

Step 4:

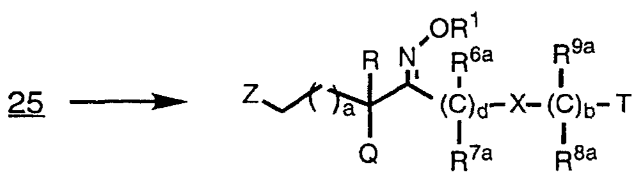

25

In step 4, compound 25 is reacted with an amine ZH in a manner similar to that of Procedure B, Step 6, to obtain the compound of formula I.

Starting materials Z-H are known in the art or are prepared by methods known in the art, for example procedures for preparing spiro-substituted azacycles were described by Ong, et al, J. Med. Chem., 26 (1983), p. 981-986 and in WO 94/29309, published December 22, 1994; substituted aryl piperazines were described in WO 96/10568, published April 11, 1996.

In the above procedures, T and Q generally are exemplified as $R^5$-phenyl and $R^4$-phenyl, respectively, but those skilled in the art will recognize that in many cases, similar procedures can be used to prepare compounds wherein T and Q are other than substituted-phenyl.

Reactive groups not involved in the above processes can be protected during the reactions with conventional protecting groups which can be removed by standard procedures after the reaction. The following Table 1 shows some typical protecting groups:

Table 1

| Group to be Protected | Group to be Protected and Protecting Group |
|---|---|
| $-COOH$ | $-COO$alkyl, $-COO$benzyl, $-COO$phenyl |
| >NH | >NCOalkyl, >NCObenzyl, >NCOphenyl, >$NCH_2OCH_2CH_2Si(CH_3)_3$ >$NC(O)OC(CH_3)_3$, >N-benzyl, >$NSi(CH_3)_3$, 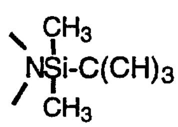 |

| Group to be Protected | Group to be Protected and Protecting Group |
| --- | --- |
| $-NH_2$ | 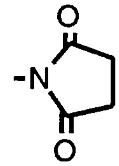 |
| $-OH$ | $-OCH_3$, $-OCH_2OCH_3$, $-OSi(CH_3)_3$, 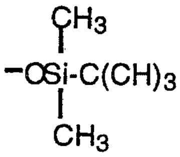 or $-OCH_2$phenyl |

Compounds of formula I have been found to be antagonists of $NK_1$ and/or $NK_2$ and/or $NK_3$ receptors, and are therefore useful in treating conditions caused or aggravated by the activity of said receptors.

The present invention also relates to a pharmaceutical composition comprising a compound of formula I and a pharmaceutically acceptable carrier. Compounds of this invention can be administered in conventional oral dosage forms such as capsules, tablets, powders, cachets, suspensions or solutions, or in injectable dosage forms such as solutions, suspensions, or powders for reconstitution The pharmaceutical compositions can be prepared with conventional excipients and additives, using well known pharmaceutical formulation techniques. Pharmaceutically acceptable excipients and additives include non-toxic and chemically compatibile fillers, binders, disintegrants, buffers, preservatives, anti-oxidants, lubricants, flavorings, thickeners, coloring agents, emulsifiers and the like.

The daily dose of a compound of formula I for treating asthma, cough, bronchspasm, inflammatory diseases, migraine, nociception and gastrointestinal disorders is about 0.1 mg to about 20 mg/kg of body weight per day, preferably about 0.5 to about 15 mg/kg. For an average body weight of 70 kg, the dosage range is therefore from about 1 to about 1500 mg of drug per day, preferably about 50 to about 200 mg, more preferably about 50 to about 500 mg/kg per day, given in a single dose or 2-4 divided doses. The exact dose, however, is determined by the attending clinician and is dependent on the potency of the compound administered, the age, weight, condition and response of the patient.

Following are examples of preparing starting materials and compounds of formula I. As used herein, Me is methyl, Ac is acetyl, Et is ethyl and Ph is phenyl.

**Preparation 1**

<u>1,2-Dihydro-1'-methyl-1-(methylsulfonyl)-spiro[3H-indole-3,4'-piperidine]</u>

Dissolve 1,2-dihydro-1'-methyl-spiro[3H-indole-3,4'-piperidine] (3.0g, 14.8 mmoles) in dry $CH_2Cl_2$ (25 ml) and cool under $N_2$ in an ice bath. Add, dropwise, methane sulfonyl chloride (1.5 ml, 19.3 mmoles), followed by ethyl diisopropyl amine (5.0 ml, 28.7 mmoles). Warm to room temperature and stir overnight. Dilute the solution to 100 ml with $CH_2Cl_2$. Wash the solution with saturated aqueous $NaHCO_3$ (4x25 ml), then with water. Dry solution with anhydrous $Na_2SO_4$, then remove the solvent using reduced pressure. Purify the resulting reaction mixture by flash chromatography ($SiO_2$) using 5% MeOH($NH_3$)/EtOAc as eluent. Yield: 3.37 g (81%) of the title compound. HRMS (FAR): calculated for $C_{14}H_{21}N_2O_2S$ $(M+H)^+$: 281.1324; found: 281.1324.

**Preparation 2**

<u>1,2-Dihydro-1-(methylsulfonyl)-spiro[3H-indole-3,4'-piperidine]</u>

Add 1-chloroethyl chlorocarbonate (1.0 ml, 9.3 mmoles) dropwise to a precooled (ice bath) solution of the product of Preparation 1 (2.0 g, 7.13 mmoles) in 1,2-dichloroethane (20 ml). After the addition, reflux the solution, under $N_2$, for 2 h. Cool the solution to room temperature and remove about half the volume of the solvent using reduced pressure. Add $CH_3OH$ (15.0 ml) and reflux the solution again for another 40 min. Remove the solvent in vacuo. Treat the resulting foam with a mixture of $CH_3OH$:EtOAc:$CH_2Cl_2$ and filter the resulting precipitate. Suspend this precipitate in $CH_2Cl_2$ (80 ml), wash 3 times with 2.5 M aqueous NaOH, then with water, and dry over anhydrous $Na_2SO_4$. Yield: 0.54 g of the title compound. Purify the filtrate by flash chromatography ($SiO_2$) using 2 1/2% $CH_3OH(NH_3)$/EtOAc as eluent to yield another 0.58 g of product. Total yield: 1.12 g (58%). HRMS (FAB): Calculated for $C_{13}H_{19}N_2O_2S$ $(M+H)^+$: 267.1167; found: 267.1174.

**Preparation 3**

1'-[5-[[3,5-Dichlorophenyl]methoxy]-3-(3,4-dichlorophenyl)-4-oxopentyl]-1,2-dihydro-spiro[3H-indole-3,4'-piperidine]

Add a solution of HBr (gas) in acetic acid (3.0 ml) to phenylmethyl 1'-[3-(3.4-dichlorophenyl)-5-[(3,5-dichlorophenyl)methoxy]-4-oxopentyl]-1,2-dihydro-spiro[3H-indole-3,4'-piperidine]-1-carboxylate [0.250 g, 0.34 mmoles) dissolved in glacial acetic acid (2.0 ml). Stir the solution at room temperature for 4 h, then add $Et_2O$ (30 ml). Decant the liquid and wash the resultant solid with $Et_2O$. Suspend the solid in water (10 ml) and add 1.0 M aqueous KOH (5.0 ml). Extract the mixture with $Et_2O$ (3x30 ml), combine the extracts, wash with water and dry ($Na_2SO_4$). Remove the solvent and purify the reaction mixture by preparative thin layer chromatography ($SiO_2$), eluting the plates twice with ETOAc:hexanes:$CH_3OH(NH_3)$ (15:85:3). Extract the desired product with 5% $CH_3OH(NH_3)$/EtOAc. Yield: 57 mg. Mass Spectrum (FAB): 591 ($^{35}Cl$ +$^{37}Cl$ isotope), 593 (M+H) ($^{35}Cl$ + $^{37}Cl$ isotope), 595 ($^{35}Cl$ + $^{37}Cl$ isotope).

**Example 1**

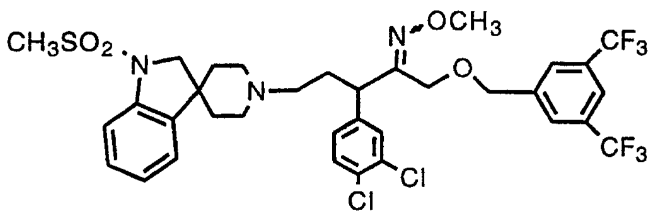

1'-[5-[[3,5-Bis(trifluoromethyl)phenyl]methoxy]-3-(3,4-dichlorophenyl)-4-(methoxyimino)pentyl]-1,2-dihydro-1-(methylsulfonyl)-spiro[3H-indole-3,4'-piperidine]

Use the method described in Procedure A to prepare the title compound. HRMS (FAB) $(M+H)^+$: calc'd for $C_{34}H_{35}N_3O_4SCl_2F_6$: 765.1630; found: 765.1610.

**Example 2**

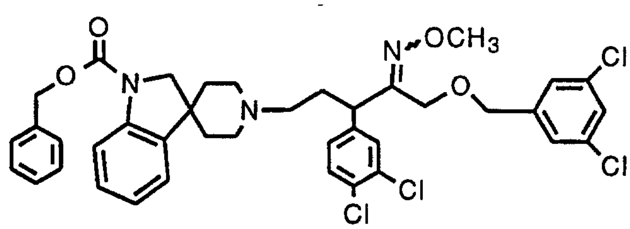

Phenylmethyl 1'-[3-(3,4-dichlorophenyl)-5-[[3,5-dichlorophenyl]methoxy]-4-(methoxyimino)pentyl]-1,2-dihydro-spiro [3H-indole-3,4'-piperidine]-1-carboxylate

Use the method described in Procedure A to prepare the title compound. HRMS (FAB) $(M+H)^+$: calc'd for $C_{39}H_{40}N_3O_4Cl_4$: 754.1773; found: 754.1757.

**Example 3**

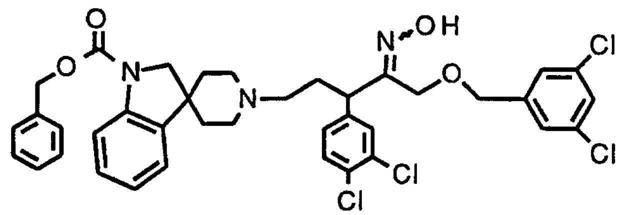

Phenylmethyl 1'-[3-(3,4-dichlorophenyl)-5-[[3,5-dichlorophenyl]methoxy]-4-(hydroxyimino)pentyl]-1,2-dihydro-spiro [3H-indole-3,4'-piperidine]-1-carboxylate

Use the method described in Procedure A to prepare the title compound. HRMS (FAB) $(M+H)^+$: calc'd for $C_{38}H_{38}N_3O_4Cl_4$: 740.1616; found: 740.1625.

**Example 4**

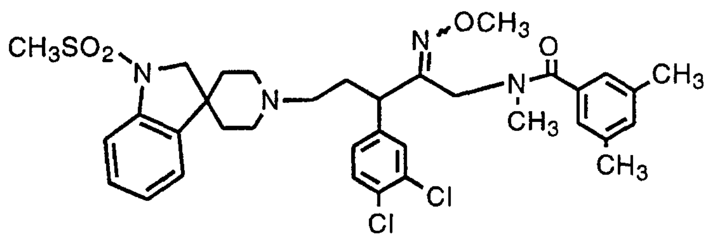

Step 1:

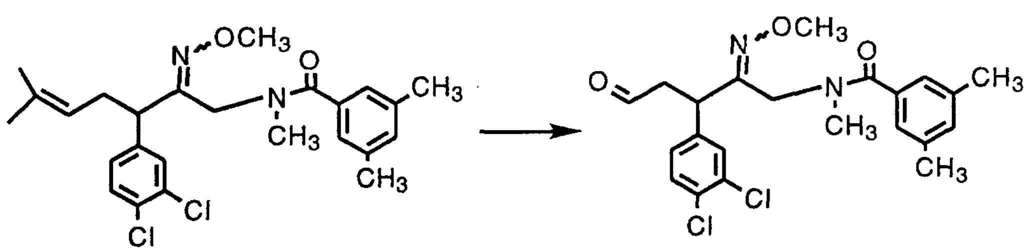

100 101

Cool a solution of 100 (10.21 g, 0.5 mmol) in EtOAc (3.6 ml) to -78°C and subject the solution to a subsurface flow of $O_2$, then $O_3$ (0.4, 1 min.), then $O_2$ for 0.5 h. Add $(CH_3)_2S$ (1 ml) to quench the reaction, remove the cold bath and allow the mixture to warm to room temperature. Wash the crude mixture with 10% $Na_2S_2O_3$, then brine, dry over $MgSO_4$, filter and concentrate to obtain a light yellow oil ($MH^+$ 435.0) to be used without further purification in the next step.

Step 2:

To a solution of the product of step 1 in $CF_3CH_2OH$ (2.6 ml), add the product of Preparation 2 (1.123 g, 0.46 mmol). When dissolution is complete, add 3Å molecular sieves (0.34 g); after 30 min., add $NaCNBH_3$ (10.17 g, 2.7 mmol). After 2 h, quench the reaction by adding deionized water (10 ml) and stir for 10 min. Partition the reaction

mixture between $Et_2O:CH_2Cl_2$ (5:1) and water. Extract the aqueous layer twice with $Et_2O:CH_2Cl_2$ (5:1), wash the combined organic layers with brine, dry over $MgSO_4$, filter and concentrate. Purify the crude product by flash chromatography and prep-TLC (twice) to obtain the title compound as a whit foam. Yield: 25.2%. MS: $MH^+$ 685.3.

**Example 5**

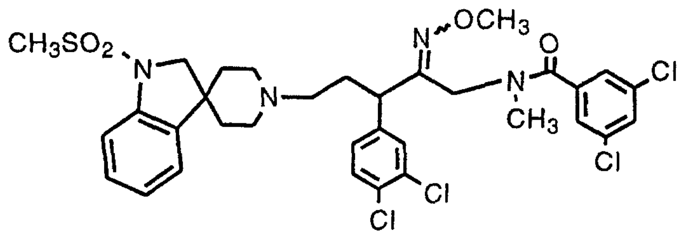

Treat a compound of the formula

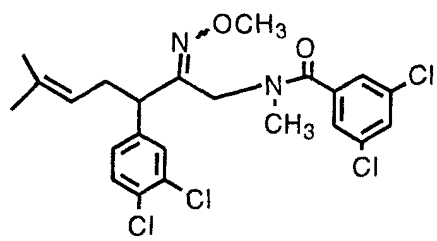

using a procedure similar to that described in Example 4 to obtain the title compound. MS (FAB) 727.2 (100%).

The following formulations exemplify some of the dosage forms of this invention. In each, the term "active compound" refers to a compound of formula I.

EXAMPLE A

| Tablets | | | |
|---|---|---|---|
| No. | Ingredient | mg/tablet | mg/tablet |
| 1 | Active Compound | 100 | 500 |
| 2 | Lactose USP | 122 | 113 |
| 3 | Corn Starch, Food Grade, as a 10% paste in Purified Water | 30 | 40 |
| 4 | Corn Starch, Food Grade | 45 | 40 |
| 5 | Magnesium Stearate | 3 | 7 |
| | Total | 300 | 700 |

Method of Manufacture

Mix Item Nos. 1 and 2 in suitable mixer for 10-15 minutes. Granulate the mixture with Item No. 3. Mill the damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weight on a suitable tablet machine.

EXAMPLE B

| Capsules | | | |
|---|---|---|---|
| No. | Ingredient | mg/tablet | mg/tablet |
| 1 | Active Compound | 100 | 500 |
| 2 | Lactose USP | 106 | 123 |
| 3 | Corn Starch, Food Grade | 40 | 70 |
| 4 | Magnesium Stearate NF | 4 | 7 |
| | Total | 250 | 700 |

Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

EXAMPLE C

| Sterile Powder for Injection | | |
|---|---|---|
| Ingredient | mg/vial | mg/vial |
| Active sterile powder | 100 | 500 |

For reconstitution add sterile water for injection or bacteriostatic water for injection.
The *in vitro* and *in vivo* activity of the compounds of formula I can be determined by the following procedures.

**In vitro procedure to identify $NK_1$ activity**

Test compounds are evaluated for their ability to inhibit the activity of the $NK_1$ agonist Substance P on the isolated guinea pig vas deferens. Freshly cut vas deferens are removed from male Hartley guinea pigs (230-350g) and suspended in 25 ml tissue baths containing Kreb's Henseleit solution warmed to 37°C and constantly aerated with 95% $O_2$ and 5% $CO_2$. Tissues are adjusted to 0.5 g and allowed to equilibrate for a period of 30 minutes. The vas deferens are exposed to an electrical field stimulation (Grass S48 Stimulator) every 60 seconds at an intensity that will cause the tissue to contract 80% of its maximum capacity. All responses are recorded isometrically by means of a Grass force displacement transducer (FT03) and Harvard electronic recorder. Substance P potentiates the electrical field stimulated-induced contractions of the guinea pig vas deferens. In unpaired studies, all tissues (control or drug treated) are exposed to cumulative concentations of Substance P ($1X10^{-10}$ M - $7X10^{-7}$ M). Single log-concentations of the test compounds are given to separate tissues and allowed to equilibrate for 30 minutes before a Substance P concentation-response curve is generated. At least 5 separate tissues are used for each control and individual drug-concentation for every drug assay.
Inhibition of the Substance P is demonstrated by a rightward shift of its concentration-response curve. These shifts are used to determine the $pA_2$ value, which is defined as the negative log of the molar concentration of the inhibitor which would require that twice as much agonist be used to elicit a chosen response. This value is used to determine relative antagonist potency.

**Isolated Hamster Trachea $NK_2$ Assay**

General methodology and characterization of hamster trachea responses to neurokinin agonists as providing an $NK_2$ monoreceptor assay is found in C.A. Maggi, et al., *Eur. J. Pharmacol.* 166 (1989) 435 and J.L. Ellis, et al., *J. Pharm. Exp. Ther.* 267 (1993) 95.
Continuous isometric tension monitoring is achieved with Grass FT-03 force displacement transducers connected to Buxco Electronics preamplifiers built into a Graphtec Linearcorder Model WR 3310.
Male Charles River LAK:LVG (SYR) hamsters, 100-200 g fed weight, are stunned by a sharp blow to the

head, loss of corneal reflex is assured, the hamsters are sacrificed by thoractomy and cutting the heart. Cervical trachea segments are removed to room temperature Krebs buffer, pH 7.4, aerated with 95% $O_2$ - 5% $CO_2$ gas and cleaned of adhering tissue. The segments are cut into two 3-4 mm long ring segments. Tracheal rings are suspended from transducers and anchored in 15.0 ml water jacketed organ baths by means of stainless steel hooks and 6-0 silk. Baths are filled with Krebs buffer, pH 7.4, maintained at 37°C and continuously aerated with 95% $O_2$ - 5% $CO_2$ gas. Tracheal rings are placed under 1.0 g initial tension and allowed a 90 min equilibration period with four 1 μM NKA challenge, wash and recovery cycles at 20 min intervals. 30 min vehicle pretreatment is followed by cumulative additions of rising doses of NKA (3 nM - 1 μM final concentration, 5 min intervals between additions). The final NKA response is followed by a 15 min wash and recovery period. 30 min pretreatment with a test compound or its vehicle is followed by cumulative additions of rising doses of NKA (3 nM - 10 μM final concentration if necessary, 5 min intervals between additions). The final NKA response is followed by a 1 mM carbachol challenge to obtain a maximal tension response in each tissue.

Tissue responses to NKA are recorded as positive pen displacements over baseline and converted to grams tension by comparison to standard weights. Responses are normalized as a % of the maximal tissue tension. $ED_{50}$'s are calculated for NKA from the control and treated NKA dose responses and compared. Test compounds resulting in an agonist dose ratio ≥ 2 at a screening concentration of 1 μM (i.e. $pA_2$ ≥ = 6.0) are considered actives. Further dose response data is obtained for actives so that an apparent $pA_2$ estimate can be calculated. $pA_2$ is calculated either by estimation of $K_i$ as described by Furchgott (where $pA_2$ = - Log $K_i$, R.F. Furchgott, *Pharm. Rev.* 7 [1995] 183) or by Shild Plot Analysis (O. Arunlakshana & H.O. Shild, *Br. J. Pharmacol.* 14[1959] 48) if the data is sufficient.

**Effect of $NK_1$ Antagonists on Substance P-Induced Airway Microvascular Leakage in Guinea Pigs**

Studies are performed on male Hartley guinea pigs ranging in weight from 400-650 g. The animals are given food and water *ad libitum*. The animals are anesthetized by intraperitoneal injection of dialurethane (containing 0.1 g/ml diallylbarbituric acid, 0.4 g/ml ethylurea and 0.4 g/ml urethane). The trachea is cannulated just below the larynx and the animals are ventilated ($V_T$ = 4 ml, f = 45 breaths/min) with a Harvard rodent respirator. The jugular vein is cannulated for the injection of drugs.

The Evans blue dye technique (Danko, G. et al., Pharmacol. Commun., 1, 203-209, 1992) is used to measure airway microvascular leakage (AML). Evans blue (30 mg/kg) is injected intravenously, followed 1 min later by i.v. injection of substance P (10 μg/kg). Five min later, the thorax is opended and a blunt-ended 13-guage needle passed into the aorta. An incision is made in the right atrium and blood is expelled by flushing 100 ml of saline through the aortic catheter. The lungs and trachea are removed en-bloc and the trachea and bronchi are then blotted dry with filter paper and weighed. Evans blue is extracted by incubation of the tissue at 37°C for 18 hr in 2 ml of formamide in stoppered tubes. The absorbance of the formamide extracts of dye is measured at 620 nm. The amount of dye is calculated by interpolation from a standard curve of Evans blue in the range 0.5-10 μg/ml in formamide. The dye concentration is expressed as ng dye per mg tissue wet weight. Test compounds were suspended in cyclodextran vehicle and given i.v. 5 min before substance P.

**Measurement of $NK_2$ Activity In Vivo**

Male Hartley guinea pigs (400-500 gm) with ad lib. access to food and water are anesthetized with an intraperitoneal injection of 0.9 ml/kg dialurethane (containing 0.1 g/m diallylbarbituric acid, 0.4 g/ml ethylurea and 0.4 g/ml urethane). After induction of a surgical plane of anesthesia, tracheal, esophageal and jugular venous cannulae are implanted to facilitate mechanical respiration, measurement of esophageal pressure and administration of drugs, respectively.

The guinea pigs are placed inside a whole body plethysmograph and the catheters connected to outlet ports in the plethysmograph wall. Airflow is measured using a differential pressure transducer (Validyne, Northridge CA, model MP45-1, range ± 2 $cmH_2O$) which measures the pressure across a wire mesh screen that covers a 1 inch hole in the wall of the plethysmograph. The airflow signal is electrically integrated to a signal proportional to volume. Transpulmonary pressure is measured as the pressure difference between the trachea and the esophagus using a differential pressure transducer (Validyne, Northridge, CA, model MP45-1, range ± 20 cm $H_2O$). The volume, airflow and transpulmonary pressure signals are monitored by means of a pulmonary analysis computer (Buxco Electronics, Sharon, CT, model 6) and used for the derivation of pulmonary resistance ($R_L$) and dynamic lung compliance ($C_{Dyn}$).

Bronchoconstriction Due to NKA

Increasing iv doses of NKA are administered at half log (0.01-3 μg/kg) intervals allowing recovery to baseline pulmonary mechanics between each dose. Peak bronchoconstriction occurs within 30 seconds after each dose of agonist. The dose response is stopped when $C_{Dyn}$ is reduced 80-90% from baseline. One dose-response to NKA is

performed in each animal. Test compounds are suspended in cyclodextran vehicle and given i.v. 5 min before the initiation of the NKA dose response.

For each animal, dose response curves to NKA are constructed by plotting the percent increase in $R_L$ or decrease in $C_{Dyn}$ against log dose of agonist. The doses of NKA that increased $R_L$ by 100% ($R_L$100) or decreased $C_{Dyn}$ by 40% ($C_{Dyn}$40) from baseline values are obtained by log-linear interpolation of the dose response curves.

<u>**Neurokinin Receptor Binding Assay(s)**</u>

Chinese Hamster ovary (CHO) cells transfected with the coding regions for the human neurokinin 1 (NK1) of the human neurokinin 2 (NK2) receptors are grown in Dulbecco's minimal essential medium supplemented with 10% fetal calf serum, 0.1 mM non-essential amino acids, 2 mM glutamine, 100units/ml of penicillin and streptomycin, and 0.8 mg of G418/ml at 37°C in a humidified atmosphere containing 5% $CO_2$.

Cells are detached from T-175 flasks with a sterile solution containing 5mM EDTA in phosphate buffered saline. Cells are harvested by centrifugation and washed in RPMI media at 40°C for 5 minutes. The pellet is resuspended inTris-HCI (pH7.4) containing 1 uM phsphoramidon and 4 ug/ml of chymostatin at a cell density of 30 x $10^6$ cells/ml. The suspension is then homogenized in a Brinkman Polytron (setting 5) for 30-45 seconds. The homogenate is centrifuged at 800 x g for 5 min at 4°C to collect unbroken cells and nuclei. The supernatant is centrifuged in a Sorvall RC5C at 19,000 rpm (44,00 x g) for 30 min at 4°C. The pellet is resuspended, an aliquot is removed for a protein determination (BCA) and washed again. The resulting pellet is stored at -80°C.

To assay receptor binding, 50 µl of [$^3$H]-Substance P (9-Sar, 11-Met [02]) (specific activity 41 Ci/mmol) (Dupont-NEN) (0.8 nM for the NK-1 assay) or [$^3$H]-Neurokinin A (specific activity 114 Ci/ mmole) (Zenca) (1.0 nM for the NK-2 assay) is added to tubes containing buffer (50 mM Tris-HCI (pH 7.4) with 1 mM $MnCl_2$ and 0.2% Bovine Serum Albumin) and either DMSO or test compound. Binding is initiated by the addition of 100µl of membrane (10-20 µg) containing the human NK-1 or NK-2 receptor in a final volume of 200 µl. After 40 minutes at room temperature, the reaction is stopped by rapid filtration onto Whatman GF/C filters which have been presoaked in 0.3% polyethylenimine. Filters are washed 2 times with 3 ml of 50 mM Tris-HCI (pH7.4). Filters are added to 6 mls of Ready-Safe liquid scintillation cocktail and quantified by liquid scintillation spectrometry in a LKB 1219 RackBeta counter. Non-specific binding is determined by the addition of either 1 µM of CP-99994 (NK-1) or 1µM SR-48968 (NK-2) (both synthesized by the chemistry department of Schering-Plough Research Institute). $IC_{50}$ values are determined from competition binding curves and Ki values are determined according to Cheng and Prusoff using the experimentally determined value of 0.8 nM for the NK-1 receptor and 2.4 nM for the NK-2 receptor.

$NK_3$ activity is determined by following a procedure similar to that described in the literature, e.g., *Molecular Pharmacol.,* <u>48</u> (1995), p. 711-716.

% Inhibition is the difference between the percent of maximum specific binding (MSB) and 100%. The percent of MSB is defined by the following equation, wherein "dpm" is disintegrations per minute:

$$\% \text{ MOD} = \frac{(\text{dpm of unknown}) - (\text{dpm of nonspecific binding})}{(\text{dpm of total binding}) - (\text{dpm of nonspecific binding})} \text{ X } 100$$

It will be recognized that compounds of formula I exhibit $NK_1$, $NK_2$ and/or $NK_3$ antagonist activity to varying degrees, e.g., certain compounds have strong $NK_1$ antagonist activity, but weaker $NK_2$ and $NK_3$ antagonist activity, while others are strong $NK_2$ antagonists, but weaker $NK_1$ and $NK_3$ antagonists. While compounds with approximate equipotency are preferred, it is also within the scope of this invention to use compounds of with unequal $NK_1/NK_2/NK_3$ antagonist activity when clinically appropriate.

Using the test procedures described above, compounds of the present invention are found to exhibit a range of activity: percent inhibition at a dosage of 1µM ranges from about 0 to about 100% inhibition of $NK_1$ and/or about 0 to about 100% inhibition of $NK_2$. Preferred are compounds having a Ki ≤100nM for the $NK_1$ receptor. Also preferred are compounds having a Ki ≤100nM for the $NK_2$ receptor. Another group of preferred compounds are those having a Ki ≤100nM for each of the $NK_1$ and $NK_2$ receptors.

**Claims**

**1.** A compound represented by the structural formula

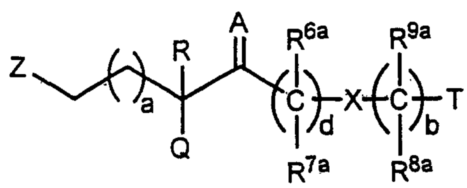

I

or a pharmaceutically acceptable salt thereof, wherein:

a is 0, 1, 2 or 3;
b and d are independently 0, 1 or 2;
R is H;
A is $=N-OR^1$;
X is a bond, -C(O)-, -O-, $-NR^6-$, $-S(O)_e-$, $-N(R^6)C(O)-$, $-OC(O)NR^6-$, $-C(=NOR^1)-$, provided that when d is 0, X is a bond, -C(O)-, $-NR^6-$, $-N(R^6)C(O)-$, $-OC(O)NR^6-$, $-C(=NOR^1)-$;
T is $R^4$-phenyl;
Q is $R^5$-phenyl;
$R^1$ is H, $C_{1-6}$ alkyl, $-(CH_2)_n-G$, $-(CH_2)_p-M-(CH_2)_n-G$, or $-C(O)N(R^6)(R^7)$;
$R^4$ and $R^5$ are independently 1-3 substituents independently selected from the group consisting of H, halogeno, $-OR^6$, $C_{1-6}$ alkyl, $-CF_3$;
$R^6$, $R^7$,and $R^8$, are independently selected from the group consisting of H, $C_{1-6}$ alkyl, $C_2$-$C_6$ hydroxyalkyl, $C_1$-$C_6$ alkoxy-$C_1$-$C_6$ alkyl, $R^{15}$-phenyl, and $R^{15}$-benzyl; or $R^6$ and $R^7$, together with the nitrogen to which they are attached, form a ring of 5 to 6 members, wherein 0, 1 or 2 ring members are selected from the group consisting of -O-, -S- and $-N(R^{19})-$;
$R^{6a}$, $R^{7a}$, and $R^{8a}$ are independently selected from the group consisting of H, $C_2$-$C_6$ hydroxyalkyl, and $C_1$-$C_6$ alkoxy-$C_1$-$C_6$ alkyl;
$R^9$ and $R^{9a}$ are independently selected from the group consisting of $R^6$;
$R^{10}$ and $R^{10a}$ are independently selected from the group consisting of H and $C_{1-6}$ alkyl;
$R^{15}$ is 1 to 3 substituents independently selected from the group consisting of H, $C_1$-$C_6$ alkyl, $C_1$-$C_6$ alkoxy, $C_1$-$C_6$ alkylthio, halogeno, $-CF_3$, $-C_2F_5$, $-COR^{10}$, $-CO_2R^{10}$, $-C(O)N(R^{10})_2$, $-S(O)_eR^{10a}$, -CN, $-N(R^{10})COR^{10}$, $-N(R^{10})CON(R^{10})_2$ and $-NO_2$;
$R^{19}$ is H, $C_1$-$C_6$ alkyl, $-C(O)N(R^{10})_2$, $-CO_2R^{10}$, $-(C(R^8)(R^9))_f-CO_2R^{10}$ or $-(C(R^8)(R^9))_u-C(O)N(R^{10})_2$;
f, n and p are independently 1-6;
u is 0-6;
G is selected from the group consisting of $R^4$-heteroaryl, $-OR^6$, $-CO_2R^6$, $-CON(R^7)(R^9)$, $-C(=NOR^8)N(R^6)(R^7)$, and $-C(O)N(R^9)-(R^4\text{-heteroaryl})$, provided that when n is 1 and u is 0, G is not -OH;
M is selected from the group consisting of -O- and $-C(O)N(R^9)-$, provided that when n is 1, G is not OH;
e is 0, 1 or 2, provided that when e is 1 or 2, $R^{10a}$ is not H;
Z is

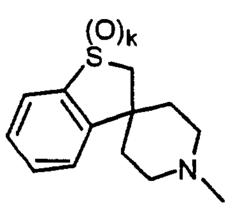 , 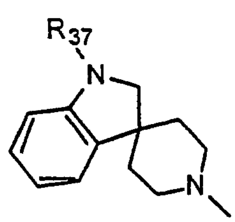 , or 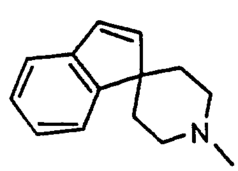 ,

$R^{37}$ is selected from a group consisting of:

(1) H,
(2) $C_{1-8}$ linear or branched alkyl, unsubstituted, monosubstituted or multiply substituted with $-OR^{35}$, =O, $-NHCOR^{35}$, $-NR^{35}R^{36}$, -CN, -halogeno,$-CF_3$, -phenyl or substituted phenyl, wherein the substitutents on phenyl are selected from the group consisting of -OH, -CN, halogeno and $-CF_3$;
(3) $S(O)R^{38}$, wherein $R^{38}$ is $C_{1-6}$ linear or branched alkyl, unsubstituted, mono di or trisubstituted with a substituent selected from the group consisting of =O, -CN, $-OR^{35}$, $-NR^{35}R^{36}$, $-NR^{36}OR^{36}$,-halogeno,$-CF_3$,

-phenyl or mono, di or trisubstituted phenyl, wherein the substituents on the phenyl are independently selected from the group consisting of -OH, =O, -CN, $-NR^{35}R^{36}$, $-NR^{35}COR^{36}$, -halogeno, $-CF_3$ and $C_{1-3}$ alkyl;
(4) $SO_2R^{38}$,
(5) $COR^{38}$,
(6) $CO_2R^{38}$;
(7) $CONR^{36}R^{38}$;

$R^{35}$ and $R^{36}$ are independently selected from:

(a) H,
(b) $C_{1-6}$ alkyl, or mono or disubstituted $C_{1-6}$ alkyl, wherein the substitutents independently selected from the group consisting of phenyl, unsubstituted or substituted with -OH, $C_{1-3}$ alkyl, -CN, halogeno, $-CF_3$ or $C_{1-4}$ alkoxy; -OH; =O; -CN; halogeno; or $-CF_3$;
(c) phenyl, pyridinyl or thiophene, or mono, di or trisubstituted phenyl, pyridinyl or thiophene, wherein the substitutents are independently selected from the group consisting of -OH, $C_{1-4}$ alkyl, -CN, halogeno and $-CF_3$;
(d) $C_{1-3}$ alkyloxy,

or
$R^{35}$ and $R^{36}$ are joined together to form a 5-, 6-, or 7-membered monocyclic saturated ring containing 1 or 2 heteroatoms independently selected from nitrogen, oxygen, and sulfur, and in which the ring is unsubstituted or mono or disubstituted, wherein the substituents are independently selected from the group consisting of -OH, =O, -CN, halogeno and $-CF_3$.

**2.** A compound of claim 1 represented by the formula

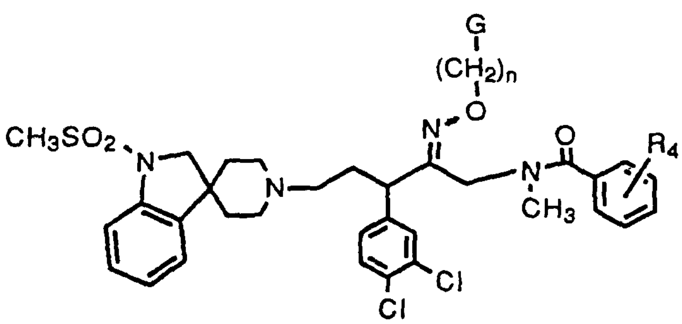

wherein G and $R_4$ are as defined in claim 1.

**3.** A compound of claim 2 represented by the structural formula

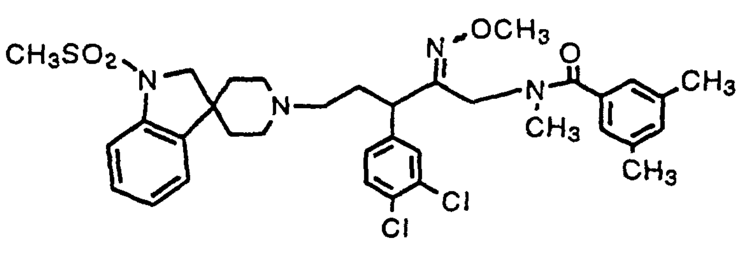

**4.** A pharmaceutical composition comprising an effective amount of a compound of any of claims1 to 3 and a pharmaceutically acceptable carrier.

**5.** The use of a compound of any of claims 1 to 3 for the preparation of a medicament for treating asthma, cough, bronchospasm, central nervous system diseases, inflammatory diseases and gastrointestinal disorders.

## Patentansprüche

**1.** Verbindung dargestellt durch die Strukturformel

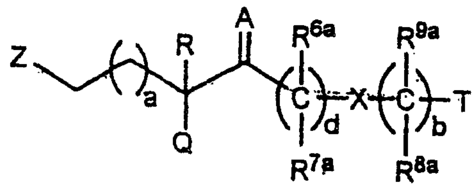

oder ein pharmazeutisch akzeptables Salz davon, wobei:

a 0, 1, 2 oder 3 ist,
b und d unabhängig voneinander 0, 1 oder 2 sind;
R H ist;
A =N-$OR^1$ ist;
X eine Bindung, -C(O)-, -O-, -$NR^6$-, -$S(O)_e$-, -$N(R^6)C(O)$-, -$OC(O)NR^6$-, -$C(=NOR^1)$- ist, vorausgesetzt dass, wenn d 0 ist, X eine Bindung, -C(O)-, -$NR^6$-, -$N(R^6)C(O)$-, -$OC(O)NR^6$-, -$C(= NOR^1)$- ist;
T $R^4$-Phenyl ist;
Q $R^5$-Phenyl ist;
$R^1$ H, $C_{1-6}$-Alkyl, -$(CH_2)_n$-G, -$(CH_2)_p$-M-$(CH_2)_n$-G oder -$C(O)N(R^6)$ $(R^7)$ ist;
$R^4$ und $R^5$ sind unabhängig voneinander 1-3 Substituenten unabhängig ausgewählt aus der Gruppe bestehend aus H, Halogen, -$OR^6$, $C_{1-6}$-Alkyl, -$CF_3$;
$R^6$, $R^7$ und $R^8$ unabhängig ausgewählt aus der Gruppe bestehend aus H, $C_{1-6}$-Alkyl, $C_2$- bis $C_6$-Hydroxyalkyl, $C_1$- bis $C_6$-Alkoxy-$C_1$- bis $C_6$-alkyl, $R^{15}$-Phenyl und $R^{15}$-Benzyl sind; oder $R^6$ und $R^7$ zusammen mit dem Stickstoff, an den sie gebunden sind, einen 5- bis 6-gliedrigen Ring bilden, wobei 0, 1 oder 2 Ringglieder aus der Gruppe bestehend aus -O-, -S- und -$N(R^{19})$- ausgewählt sind,
$R^{6a}$, $R^{7a}$ und $R^{8a}$ unabhängig voneinander ausgewählt sind aus der Gruppe bestehend aus H, $C_2$- bis $C_6$-Hydroxyalkyl und $C_1$bis $C_6$-Alkoxy-$C_1$- bis $C_6$-alkyl;
$R^9$ und $R^{9a}$ unabhängig voneinander ausgewählt sind aus der Gruppe bestehend aus $R^6$;
$R^{10}$ und $R^{10a}$ unabhängig voneinander ausgewählt sind aus der Gruppe bestehend aus H und $C_{1-6}$-Alkyl;
$R^{15}$ für 1 bis 3 Substituenten steht, die unabhängig voneinander ausgewählt sind aus der Gruppe bestehend aus H, $C_1$bis $C_6$-Alkyl, $C_1$- bis $C_6$-Alkoxy, $C_1$- bis $C_6$-Alkylthio, Halogen, -$CF_3$, -$C_2F_5$, -$COR^{10}$, -$CO_2R^{10}$, -C(O) $N(R^{10})_2$, -$S(O)_eR^{10a}$, -CN, -N $(R^{10})COR^{10}$, -$N(R^{10})CON(R^{10})_2$ und -$NO_2$;
$R^{19}$ H, $C_1$- bis $C_6$-Alkyl, -$C(O)N(R^{10})_2$, -$CO_2R^{10}$, -$(C(R^8)(R^9))_f$-$CO_2R^{10}$ oder -$(C(R^8)(R^9))_u$-$C(O)N(R^{10})_2$ ist,
f, n und p unabhängig voneinander 1 bis 6 sind;
u 0 bis 6 ist,
G ausgewählt ist aus der Gruppe bestehend aus $R^4$-Heteroaryl, -$OR^6$, -$CO_2R^6$, -$CON(R^7)$ $(R^9)$, -$C(=NOR^8)N$ $(R^6)$ $(R^7)$ und -$C(O)N(R^9)$-($R^4$-Heteroaryl), vorausgesetzt dass, wenn n 1 und u 0 ist, G nicht OH ist;
M ausgewählt ist aus der Gruppe bestehend aus -O- und -$C(O)N(R^9)$-, vorausgesetzt dass, wenn n 1 ist, G nicht OH ist;
e 0, 1 oder 2 ist, vorausgesetzt dass, wenn e 1 oder 2 ist, $R^{10a}$ nicht H ist;
Z

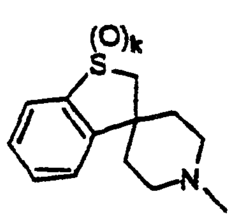

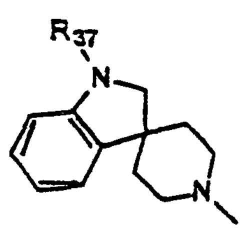

oder

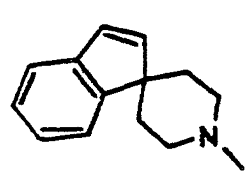

ist; $R^{37}$ ausgewählt ist aus der Gruppe bestehend aus:

(1) H,

(2) Lineares oder verzweigtes $C_{1-8}$- Alkyl, unsubstituiert, monosubstituiert oder mehrfachsubstituiert mit $-OR^{35}$, =O, $-NHCOR^{35}$, $-NR^{35}R^{36}$, -CN, -Halogen, $-CF_3$, -Phenyl oder substituiertem Phenyl, wobei die Substituenten am Phenyl ausgewählt sind aus der Gruppe bestehend aus -OH, -CN, Halogen oder $-CF_3$;
(3) $S(O)R^{38}$, wobei $R^{38}$ ein lineares oder verzweigtes $C_{1-6}$-Alkyl ist, unsubstituiert, mono, di- oder trisubstituiert mit einem Substituenten ausgewählt aus der Gruppe bestehend aus =O, -CN, $-OR^{35}$, $-NR^{35}R^{36}$, $-NR^{36}OR^{36}$, -Halogen, $-CF_3$, -Phenyl oder mono-, di oder trisubstituiertem Phenyl, wobei die Substituenten an dem Phenyl unabhängig ausgewählt sind aus der Gruppe bestehend aus -OH, =O, -CN, $-NR^{35}R^{36}$, $-NR^{35}COR^{36}$, Halogen, $-CF_3$ und $C_{1-3}$-Alkyl;
(4) $SO_2R^{38}$;
(5) $COR^{38}$;
(6) $CO_2R^{38}$;
(7) $CONR^{36}R^{38}$;

$R^{35}$ und $R^{36}$ unabhängig voneinander ausgewählt sind aus:

(a) H,
(b) $C_{1-6}$-Alkyl oder mono- oder disubstituiertem $C_{1-6}$-Alkyl, wobei die Substituenten unabhängig ausgewählt sind aus der Gruppe bestehend aus Phenyl, welches unsubstituiert oder substituiert mit -OH, $C_{1-3}$-Alkyl, -CN, Halogen, $-CF_3$ oder $C_{1-4}$-Alkoxy ist; -OH; =O; -CN; Halogen oder $-CF_3$;
(c) Phenyl, Pyridinyl oder Thiophen, oder mono-, dioder trisubstituiertes Phenyl, Pyridinyl oder Thiophen, wobei die Substituenten unabhängig ausgewählt sind aus der Gruppe bestehend aus -OH, $C_{1-4}$-Alkyl, -CN, Halogen und $-CF_3$;
(d) $C_{1-3}$-Alkoxy,

oder
$R^{35}$ und $R^{36}$ miteinander verbunden sind, um einen 5-, 6-, oder 7-gliedrigen monocyclischen gesättigten Ring zu bilden, welcher ein oder zwei Heteroatome enthält, die unabhängig ausgewählt sind aus Stickstoff, Sauerstoff und Schwefel, und wobei der Ring unsubstituiert oder mono- oder disubstituiert ist, wobei die Substituenten unabhängig ausgewählt sind aus der Gruppe bestehend aus -OH, =O, -CN, Halogen und $-CF_3$.

**2.** Verbindung nach Anspruch 1 dargestellt durch die Formel

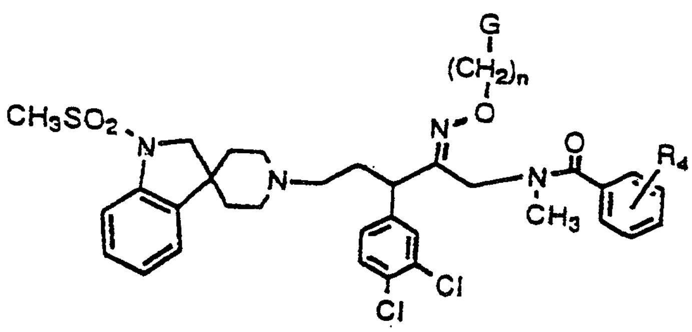

wobei G und $R_4$ wie in Anspruch 1 definiert sind.

**3.** Verbindung nach Anspruch 2, die durch die Strukturformel

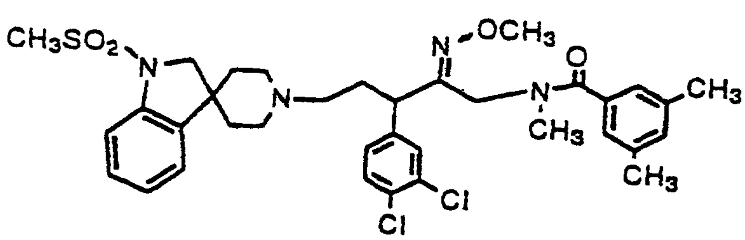

dargestellt ist.

4. Pharmazeutische Zusammensetzung, die eine wirksame Menge einer Verbindung nach einem der Ansprüche 1 bis 3 und einen pharmazeutisch akzeptablen Träger enthält.

5. Verwendung einer Verbindung nach einem der Ansprüche 1 bis 3 für die Herstellung eines Medikaments zur Behandlung von Asthma, Husten, Bronchospasmen, Krankheiten des zentralen Nervensystems, Entzündungskrankheiten und gastrointestinalen Störungen.

## Revendications

1. Composé représenté par la formule structurale ;

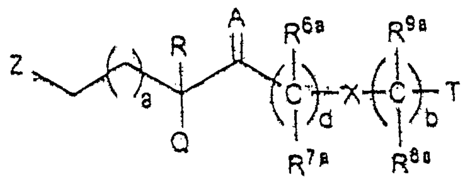

dans laquelle ;

| | |
|---|---|
| a | est égal à 0, 1, 2 ou 3, |
| b | et d représentent chacun indépendamment 0, 1 ou 2, |
| R | est H, |
| A | est =N-OR$^1$, |
| X | désigne une liaison simple, -C(O)-, -O-, -NR$^6$-, -S(O)$_e$-, -N(R$^6$)C(O)-, -OC(O)NR$^6$-, ou -C(=NOR$^1$)-, étant entendu que lorsque d est égal à 0, X est une liaison simple, -C(O)-, -NR$^6$-, -N(R$^6$)C(O)-, -OC(O)NR$^6$- ou -C(=NOR$^1$)-, |
| T | représente un groupe R$^4$-phényle, |
| Q | représente un groupe R$^5$-phényle, |
| R$^1$ | représente H, un groupe alkyle en $C_1$ à $C_6$, -(CH$_2$)$_n$-G, - (CH$_2$)$_p$-M-(CH$_2$)$_n$-G ou -C(O)N(R$^6$) (R$^7$), |
| R$^4$ et R$^5$ | représentent chacun indépendamment 1 à 3 substituants pris parmi H, les atomes d'halogène, -OR$^6$, les groupes alkyle en $C_1$ à $C_6$ et -CF$_3$, |
| R$^6$, R$^7$ et R$^8$ | représentent chacun indépendamment H ou un groupe alkyle en $C_1$ à $C_6$, hydroxyalkyle en $C_2$ à $C_6$, alcoxy($C_1$ à $C_6$) alkyle($C_1$ à $C_6$), R$^{15}$-phényle ou R$^{15}$-benzyle, ou bien |
| R$^6$ et R$^7$ | forment avec l'atome d'azote auquel ils sont liés un cycle à 5 ou 6 chaînons, 0, 1 ou 2 éléments du cycle étant choisis parmi -O-, -S- et -N(R$^{19}$)-, |
| R$^{6a}$, R$^{7a}$ et R$^{8a}$ | représentent chacun indépendamment H, un groupe hydroxyalkyle en $C_2$ à $C_6$ ou un groupe alcoxy($C_1$ à $C_6$)-alkyle($C_1$ à $C_6$), |
| R$^9$ et R$^{9a}$ | représentent chacun indépendamment R$^6$, |
| R$^{10}$ et R$^{10a}$ | représentent chacun indépendamment H ou un groupe alkyle en $C_1$ à $C_6$, |
| R$^{15}$ | désigne 1 à 3 substituants choisis indépendamment parmi H, les groupes alkyle en $C_1$ à $C_6$, les groupes alcoxy en $C_1$ à $C_6$, les groupes alkylthio en $C_1$ à $C_6$, les atomes d'halogène, -CF$_3$, -C$_2$F$_5$, -COR$^{10}$, -CO$_2$R$^{10}$, -C(O)N(R$^{10}$)$_2$, -S(O)$_e$R$^{10a}$, -CN, -N(R$^{10}$)COR$^{10}$, -N(R$^{10}$)CON(R$^{10}$)$_2$ et -NO$_2$, |
| R$^{19}$ | représente H, un groupe alkyle en $C_1$ à $C_6$, -C(O)N(R$^{10}$)$_2$, -CO$_2$R$^{10}$, - (C(R$^8$) (R$^9$))$_f$ -CO$_2$R$^{10}$ ou - (C (R$^8$) (R$^9$))$_u$-C(O) N (R$^{10}$)$_2$, |
| f, n et p | représentent chacun indépendamment un nombre de 1 à 6, |
| u | représente un nombre de 0 à 6, |
| G | représente un groupe R$^4$-hetéroaryle, -OR$^6$, -CO$_2$R$^6$, -CON(R$^7$) (R$^9$), -C(=NOR$^8$)N(R$^6$) (R$^7$) ou -C(O)N(R$^9$)-(R$^4$-hétéroaryle), étant entendu que lorsque n est égal à 1 et u est égal à 0, G n'est pas -OH, |
| M | représente -O- ou -C(O)N(R$^9$)-, étant entendu que lorsque n est égal à 1, G n'est pas OH, |
| e | désigne 0, 1 ou 2, étant entendu que lorsque e est égal à à 1 ou 2, R$^{10a}$ n'est pas H, |
| Z | représente un groupe de formule : |

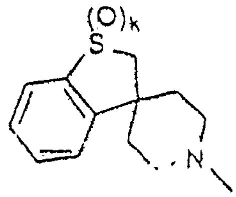 , 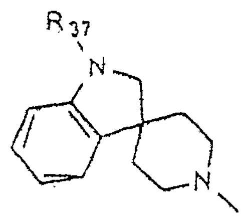 , ou 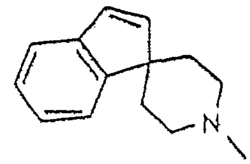 ,

dans laquelle $R^{37}$ représente ;

(1) H,
(2) un groupe alkyle linéaire ou ramifié en $C_1$ à $C_8$, non substitué ou monosubstitué ou multisubstitué par un ou plusieurs substituants pris parmi $-OR^{35}$, =O, $-NHCOR^{35}$, $-NR^{35}R^{36}$, -CN, les atomes d'halogène, $-CF_3$, le groupe phényle et le groupe phényle substitué, les substituants du groupe phényle étant choisis parmi -OH, -CN, les atomes d'halogène et $-CF_3$,
(3) $S(O)R^{38}$ dans lequel $R^{38}$ désigne un groupe alkyle linéaire ou ramifié en $C_1$ à $C_6$, non substitué ou mono-, di- ou trisubstitué par un ou plusieurs substituants pris parmi =O, -CN, $-OR^{35}$, $-NR^{35}R^{36}$, $-NR^{36}OR^{36}$, les atomes d'halogène, $-CF_3$, le groupe phényle et le groupe phényle monosubstitué, disubstitué ou trisubstitué, le ou les substituants du groupe phényle étant choisis indépendamment parmi -OH, =O, -CN, $-NR^{35}R^{36}$, $-NR^{35}COR^{36}$, les atomes d'halogène, $-CF_3$ et les groupes alkyle en $C_1$ à $C_3$,
(4) $SO_2R^{38}$,
(5) $COR^{38}$,
(6) $CO_2R^{38}$, ou
(7) $CONR^{36}R^{38}$,

$R^{35}$ et $R^{36}$ représentent chacun indépendamment

(a) H,
(b) un groupe alkyle en $C_1$ à $C_6$ ou un groupe alkyle en $C_1$ à $C_6$ monosubstitué ou disubstitué, le ou les substituants étant choisis indépendamment parmi -OH, =O, -CN, les atomes d'halogène, $-CF_3$, et le groupe phényle, non substitué ou substitué par -OH, un groupe alkyle en $C_1$ à $C_3$, -CN, un atome d'halogène, $-CF_3$ ou un groupe alcoxy en $C_1$ à $C_4$,
(c) un groupe phényle, pyridinyle ou thiophène, non substitué ou mono-, di- ou trisubstitué, le ou les substituants étant choisis indépendamment parmi -OH, les groupes alkyle en $C_1$ à $C_4$, -CN, les atomes d'halogène et $-CF_3$, ou
(d) un groupe alcoxy en $C_1$ à $C_3$,

ou bien $R^{35}$ et $R^{36}$ sont réunis pour former un cycle saturé monocyclique à 5, 6 ou 7 chaînons, contenant 1 ou 2 hetéroatomes choisis indépendamment parmi les atomes d'azote, d'oxygène et de soufre, ledit cycle étant non substitué ou mono- ou disubstitué, le ou les substituants étant choisis indépendamment parmi -OH, =O, -CN, les atomes d'halogène et $-CF_3$,

ou un sel pharmaceutiquement acceptable d'un tel composé.

**2.** Composé selon la revendication 1, qui est représenté par la formule :

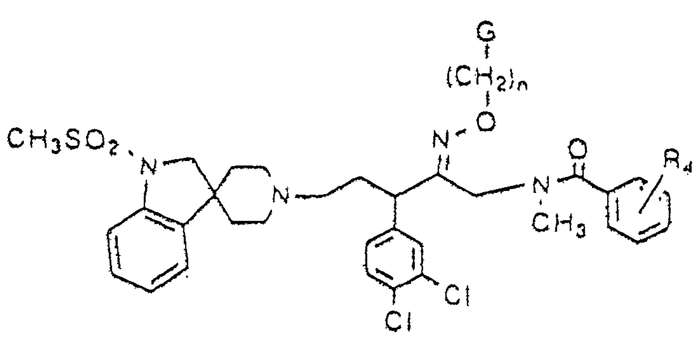

dans laquelle G et $R_4$ sont tels que définis dans la revendication 1.

**3.** Composé selon la revendication 2, qui est représenté par le formule structurale :

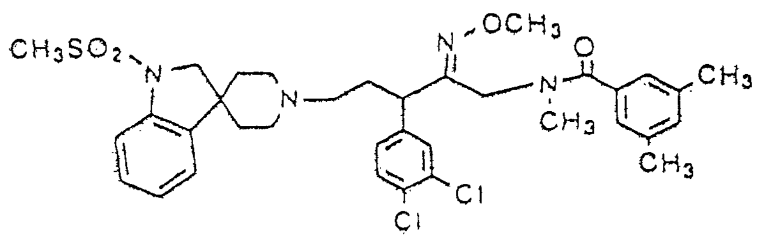

**4.** Composition pharmaceutique comprenant une quantité efficace d'un composé selon l'une quelconque des revendications 1 à 3 et un support pharmaceutiquement acceptable.

**5.** Utilisation d'un composé selon l'une quelconque des revendications 1 à 3 pour la préparation d'un médicament destiné au traitement de l'asthme, de la toux, des spasmes bronchiques, des maladies du système nerveux central, des maladies inflammatoires et des désordres gastro-intestinaux.